# EUROPEAN PATENT APPLICATION

(11) **EP 4 721 738 A1**
(43) Date of publication of application: **08.04.2026**
(21) Application number: 23938771.5
(22) Date of filing: 29.05.2023
(51) Int. Cl.: A61K 31/4375, A61P 31/04

(54) **JOINT CAVITY DRUG ADMINISTRATION METHOD, AND USE THEREOF**

(71) Applicant: TenNor Therapeutics (Suzhou) Limited, Suzhou, Jiangsu 215123 (CN)
(72) Inventor: MA, Zhenkun, Suzhou, Jiangsu 215123 (CN); WANG, Huan, Suzhou, Jiangsu 215123 (CN); GENG, Guozhu, Suzhou, Jiangsu 215123 (CN)
(74) Representative: Weickmann & Weickmann PartmbB
(86) International application number: PCT/CN2023/096858
(87) International publication number: WO 2024/243773

(57) **Abstract**

The present application provides a method for preventing or treating a prosthetic joint infection (PJI), or for alleviating PJI-related symptoms. The method comprises: administering a therapeutic agent to a joint cavity of a patient in need thereof, the therapeutic agent comprising a compound represented by formula (I) and/or a pharmaceutically acceptable salt, a hydrate, a solvate, a prodrug, a metabolite, or a deuterated substance thereof.

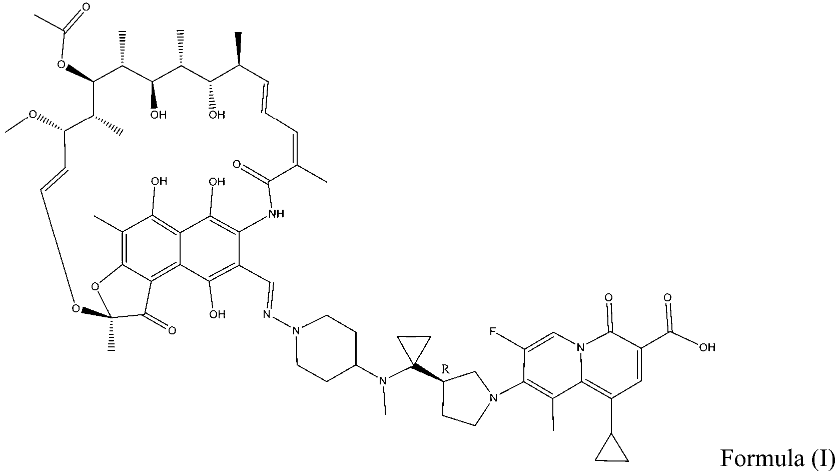

## Description

### TECHNICAL FIELD

The present application relates to the field of biomedicine, in particular to a method for preventing or treating a disease by administration via the joint cavity.

### BACKGROUND

Artificial arthroplasty, which involves surgically implanting a prosthesis to replace joints that have become painful and unable to function normally due to arthritis, joint diseases, osteoarthritis, rheumatoid arthritis, *etc.,* is being widely used in clinical practice as an effective treatment method. Prosthetic joint infections (PJIs) are serious complications following total hip arthroplasty (THA) and total knee arthroplasty (TKA). It may lead to complete surgical failure, cause joint dysfunction of the affected limb, and in severe cases, even result in amputation or death. The pathogenesis of PJIs is mainly associated with biofilm infections, with incidence rates of 0.5%-1.2% and 0.5%-1.6%, respectively. Furthermore, as the number of patients receiving arthroplasty increases, the number of PJI patients is showing an upward trend. The current management of PJIs usually involves a combination of surgical intervention and antimicrobial therapy. The standard surgical approach entails debridement to physically disrupt or remove the biofilm. However, this strategy often fails to prevent recurrent infections, leading to poor long-term prognoses and significant patient morbidity. Additionally, adjunctive antibiotic therapy is faced with the infectious bacteria being prone to the development of bacterial resistance and multidrug-resistant organisms. Due to the complex pathology of PJIs, the treatment is quite challenging. Patients often require multiple surgical revisions and long-term, systemic antibiotic therapy. The patients are repeatedly hospitalized and experience prolonged mobility impairment, which severely affects their quality of life. Moreover, medical costs are several times or even dozens of times higher than those for patients without PJIs. Furthermore, no matter what kind of PJI treatment regimen is adopted, the use of antibiotics is indispensable, but the medical community still lacks unified implementation standards or research conclusions regarding the timing of use, the optimal application course and dose, the route of administration, and the like of antibiotics during PJI treatment, and the clinical antibiotic use regimens are mostly based on expert consensus or experience. Therefore, there is an urgent need in the art for a (non-surgical) antibacterial treatment method that can provide relatively high quality and definite effectiveness.

### SUMMARY

In order to overcome the shortcomings of the prior art in the treatment of prosthetic joint infections, the present application provides a more practical and effective treatment method.

In one aspect, the present application provides a method for preventing or treating prosthetic joint infections (PJI) or alleviating PJI-related symptoms, which comprises administering a therapeutic agent to a joint cavity of a patient in need, wherein the therapeutic agent comprises a compound of formula (I) and/or a pharmaceutically acceptable salt, a hydrate, a solvate, a prodrug, a metabolite, or a deuteride thereof,

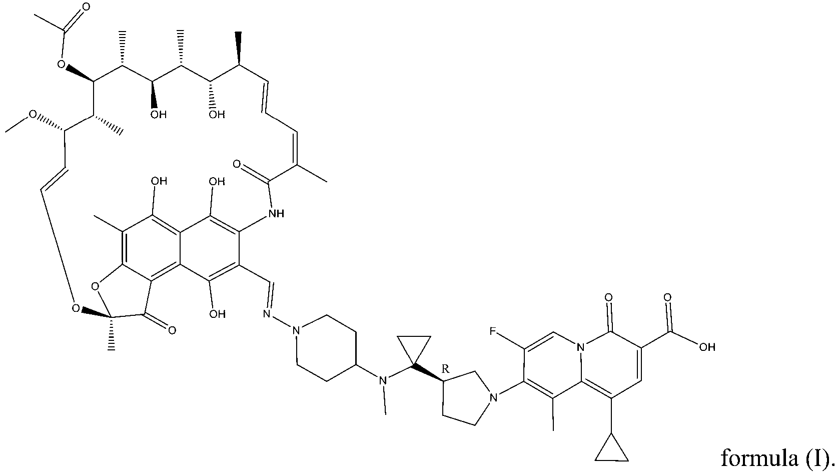

In some embodiments, the method comprises administering the therapeutic agent to the joint cavity of the patient in need at a dosing frequency optionally selected from: once daily, once every 2 days, once every 3 days, once every 4 days, once every 5 days, once every 6 days, and once every 7 days.

In some embodiments, the method comprises administering the therapeutic agent to the joint cavity of the patient in need for a duration of 1 day to about 6 months, wherein during the duration of 1 day to about 6 months, the dosing frequency of the therapeutic agent remains the same or is changed.

In some embodiments, in a single administration, the therapeutic agent is administered to the patient in need via one or more intraarticular injections.

In some embodiments, in a single administration, the therapeutic agent comprises about 10 mg to about 300 mg of the compound of formula (I) and/or the pharmaceutically acceptable salt, the hydrate, the solvate, the prodrug, the metabolite, or the deuteride thereof.

In some embodiments, the therapeutic agent further comprises additional components selected from other drugs, pharmaceutically acceptable carriers, solvents, and buffers.

In some embodiments, the additional components are not mixed with the compound of formula (I) and/or the pharmaceutically acceptable salt, the hydrate, the solvate, the prodrug, the metabolite, or the deuteride thereof.

In some embodiments, one or more of the additional components are mixed with the compound of formula (I) and/or the pharmaceutically acceptable salt, the hydrate, the solvate, the prodrug, the metabolite, or the deuteride thereof.

In some embodiments, the method further comprises administering an effective amount of an additional therapeutic agent to the patient in need.

In some embodiments, the prosthetic joint infections (PJI) are caused by Gram-positive bacteria.

In some embodiments, the prosthetic joint infections (PJI) are caused by *Staphylococcus aureus* and/or coagulase-negative staphylococcal.

In some embodiments, the prosthetic joint infections (PJI) comprise early PJI, acute hematogenous PJI, and chronic PJI.

In some embodiments, the prosthetic joint infections are early PJI occurring within about 3 months after the patient in need undergoes a prosthetic joint replacement surgery, or acute hematogenous PJI occurring within about 4 weeks after the surgery.

In some embodiments, the patient in need includes a subject who has not yet developed the prosthetic joint infections (PJI) but is at risk of developing the PJI.

In some embodiments, the method comprises administering the therapeutic agent to the joint cavity of the subject for a duration of 1 day to 14 days.

In some embodiments, the first day of the duration of 1 day to 14 days is the day on which the subject undergoes the prosthetic joint replacement surgery.

In another aspect, the present application provides use of the compound of formula (I) and/or the pharmaceutically acceptable salt, the hydrate, the solvate, the prodrug, the metabolite, or the deuteride thereof in the preparation of a therapeutic agent, wherein the therapeutic agent is used for preventing or treating prosthetic joint infections (PJI) or alleviating PJI-related symptoms, and the therapeutic agent is formulated to be suitable for intraarticular administration to a patient in need.

In some embodiments, the therapeutic agent is formulated to be suitable for administration to the joint cavity of the patient in need with a dosing frequency selected from: once daily, once every 2 days, once every 3 days, once every 4 days, once every 5 days, once every 6 days, and once every 7 days.

In some embodiments, the therapeutic agent is formulated to be suitable for administration to the joint cavity of the patient in need for a duration of 1 day to about 6 months, wherein during the duration of 1 day to about 6 months, the therapeutic agent is formulated to be suitable for administration to the patient in need at the same or different dosing frequencies.

In some embodiments, in a single administration, the therapeutic agent is formulated to be suitable for one or more intraarticular injections.

In some embodiments, in a single administration, the therapeutic agent comprises about 10 mg to about 300 mg of the compound of formula (I) and/or the pharmaceutically acceptable salt, the hydrate, the solvate, the prodrug, the metabolite, or the deuteride thereof.

In some embodiments, the therapeutic agent further comprises additional components selected from other drugs, pharmaceutically acceptable carriers, solvents, and buffers.

In some embodiments, the additional components are not mixed with the compound of formula (I) and/or the pharmaceutically acceptable salt, the hydrate, the solvate, the prodrug, the metabolite, or the deuteride thereof.

In some embodiments, one or more of the additional components are mixed with the compound of formula (I) and/or the pharmaceutically acceptable salt, the hydrate, the solvate, the prodrug, the metabolite, or the deuteride thereof.

In some embodiments, the therapeutic agent is formulated to be suitable for co-administration with an effective amount of an additional therapeutic agent to the patient in need.

In some embodiments, the prosthetic joint infections (PJI) are caused by Gram-positive bacteria.

In some embodiments, the prosthetic joint infections (PJI) are caused by *Staphylococcus aureus* and/or coagulase-negative *staphylococcal.*

In some embodiments, the prosthetic joint infections (PJI) comprise early PJI, acute hematogenous PJI, and chronic PJI.

In some embodiments, the prosthetic joint infections are early PJI occurring within about 3 months after the patient in need undergoes an artificial prosthetic joint replacement surgery, or acute hematogenous PJI occurring within about 4 weeks after the surgery.

In some embodiments, the patient in need comprises a subject who has not yet developed the prosthetic joint infections (PJI) but is at risk of developing the PJI.

In some embodiments, the therapeutic agent is formulated to be suitable for administration to the joint cavity of the subject for a duration of 1 day to 14 days.

In some embodiments, the first day of the duration of 1 day to 14 days is the day on which the subject undergoes the prosthetic joint replacement surgery.

### BRIEF DESCRIPTION OF THE DRAWINGS

The specific features of the invention to which the present application relates are set forth in the appended claims. The features and advantages of the invention to which the present application relates may be more fully understood with reference to the exemplary embodiments and drawings described in detail below. The drawings are briefly described below:

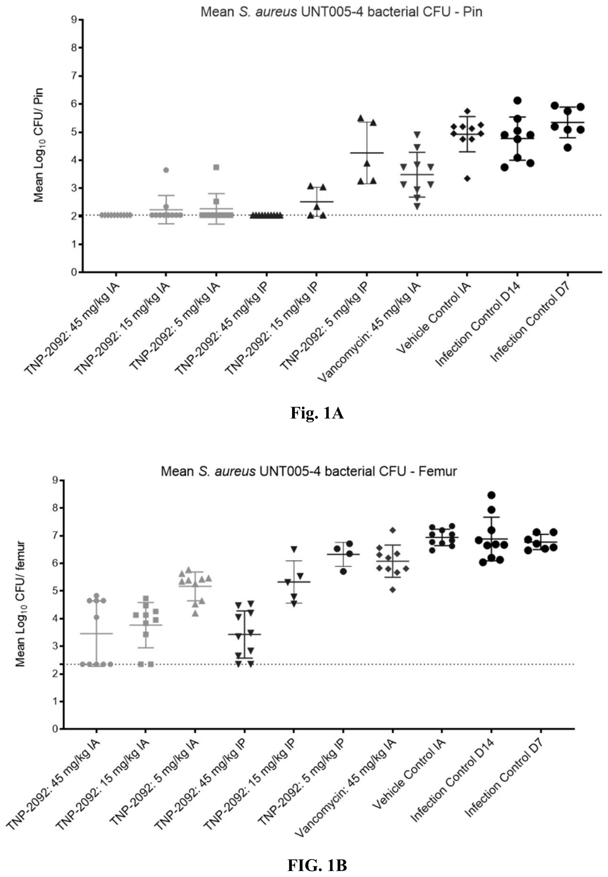

FIG. 1A-1B show the colony counts in metal implants and bone joints of mice, respectively, after administration of the treatment regimen of the present application and other treatment regimens, where the horizontal coordinates, from left to right, correspond to: IA groups of high-, medium- and low-dose of the compound of formula (I); IP groups of high-, medium- and low-dose of the compound of formula (I); an IA group of vancomycin; a vehicle control group; a blank group with 14-day infection; and a blank group with 7-day infection.

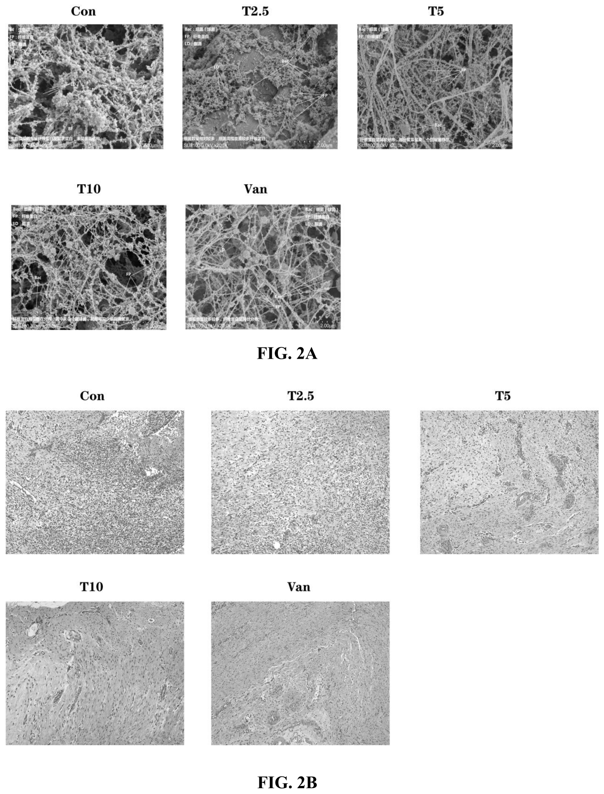

FIG. 2A-2B show the status of the implanted joint prosthesis in rats under scanning electron microscopy observation (A) and the pathological H&E staining results of the synovial soft tissue in rats (B) after administration of the treatment regimen of the present application and other treatment regimens.

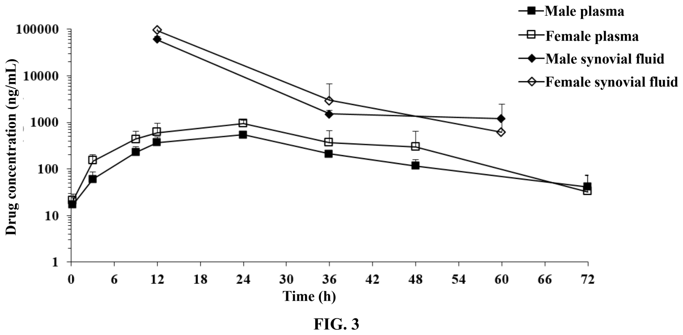

FIG. 3 shows the drug concentration-time curves of the compound of formula (I) in plasma and synovial fluid samples after administration of the treatment regimen of the present application to male and female beagle dogs.

### DETAILED DESCRIPTION

The implementation of the invention of the present application is described below with reference to specific embodiments, and those skilled in the art can easily understand other advantages and effects of the invention of the present application from the disclosure of this specification.

### Definitions of Terms

In the present application, the term "patient in need" may generally include human patients and other mammalian subjects receiving prophylactic or therapeutic treatment, including but not limited to non-human primates, laboratory animals such as rabbits, canines, rats, and mice, as well as other animals. The "patient in need" may illustratively be a subject diagnosed with a specific disease, a subject receiving treatment related to a specific disease, a subject with a tendency or risk of developing a specific disease, *etc.*

In the present application, the term "subject at risk of..." generally refers to an observed or treated individual who has a higher probability of suffering from a specific disease or disorder based on a certain risk factor. For example, in the present application, the risk factor may include, but is not limited to, lack of resistance to a specific infection or infectious disease, and previous, current, or planned exposure to an environment with infectious conditions. In certain embodiments, such subjects may be asymptomatic, or may exhibit symptoms that have not been formally confirmed.

In the present application, the term "administer", "administering", or "administration" generally refers to introducing the drug or a composition comprising the drug into a patient's body via any route of introduction or delivery. Any method known to those skilled in the art for bringing a cell, an organ, or a tissue into contact with the drug or the composition thereof can be employed. The term "single administration" may be divided into one, two, or more doses of a suitable form to be administered at one, two, or more time points during a certain period of time.

In the present application, the term "therapeutic agent" generally refers to a compound, mixture of compounds, compositions, biological macromolecules, or extracts made from biological materials that, when properly administered to a patient, is capable of inducing a desired therapeutic effect, preventing the occurrence of a certain disorder, or alleviating an associated disorder.

In the present application, the term "pharmaceutically acceptable salt" generally refers to a conventional acid- or base-addition salt that retains the biological efficacy and properties of the parent compound, for example, the compound of formula (I). By way of example only, acid-addition salts may be formed from suitable non-toxic organic or inorganic acids. Exemplary acid-addition salts include those derived from inorganic acids such as hydrochloric acid, hydrobromic acid, hydroiodic acid, sulfuric acid, sulfamic acid, phosphoric acid, nitric acid, etc., and those derived from organic acids such as p-toluenesulfonic acid, naphthalenesulfonic acid, naphthalenedisulfonic acid, methanesulfonic acid, ethanesulfonic acid, trifluoroacetic acid, etc. Base-addition salts include those derived from inorganic bases, such as sodium salts, potassium salts, lithium salts, ammonium salts, calcium salts, and magnesium salts, and those derived from organic bases, such as salts of primary amines, secondary amines, and tertiary amines. Converting (pharmaceutical) compounds into salts through chemical modification is a well-known technique for pharmaceutical chemists to achieve improved physical and chemical stability, hygroscopicity, flowability, and solubility of the compounds. See, for example, Ansel, H., et al., Pharmaceutical Dosage Forms and Drug Delivery Systems, 6th ed., 1995.

In the present application, the term "solvate" generally refers to an association compound or complex of one or more solvent molecules with the salt form of the compound of the present application. Examples of solvents for forming solvates include, but are not limited to, water, isopropanol, ethanol, methanol, DMSO, ethyl acetate, acetic acid, and ethanolamine. The term "hydrate" generally refers to a complex in which the solvent molecule is water.

In the present application, the term "prodrug" generally refers to a prodrug substance that, after administration to a patient, can be metabolized to form a substance having the structure of the compound of the present application; the term "metabolite" generally refers to a substance obtained after the compound of the present application is administered to a patient and subsequently metabolized. Such derivatives, including prodrugs and metabolites, are included within the scope of the invention of the present application.

In the present application, the terms "deuteride" and "deuterated compound" are used interchangeably and generally refer to a novel compound obtained by replacing one or more hydrogen atoms in an organic compound with deuterium atoms.

In the present application, the term "drug" generally refers to a molecule that possesses a desired biological effect. Drugs may be prophylactic or therapeutic. Drugs may include, but are not limited to: protein molecules, including but not limited to peptides, polypeptides, proteins (including post-translationally modified proteins, fusion proteins, antibodies, *etc*.); small molecules, including inorganic or organic compounds; nucleic acid molecules, including but not limited to double-stranded or single-stranded DNA, or double-stranded or single-stranded RNA (*e.g.,* antisense (molecules), RNAi, *etc*.)*,* intron sequences, triplex nucleic acid molecules, and aptamers; or vaccines (e.g., Listeria-based and non-Listeria-based vaccines). Drugs can be obtained from any known organism (including but not limited to: animals, plants, bacteria, fungi and protozoa or viruses) or from synthetic molecular libraries.

In the present application, the term "pharmaceutically acceptable carrier" generally refers to one or more non-toxic materials that do not interfere with the effectiveness of the biological activity of the active ingredient. Such formulations may routinely contain a salt, a buffering agent, a preservative, a compatible carrier, and optionally an additional therapeutic agent. Such pharmaceutically acceptable formulations may also comprise a compatible solid or liquid filler, diluent or encapsulating substance which is suitable for administration to a human. Other contemplated carriers, excipients, and/or additives that can be used in the formulations described herein may include, for example, flavoring agents, antimicrobial agents, sweeteners, antioxidants, antistatic agents, lipids, protein excipients (*e.g*., serum albumin, gelatin, and casein), salt-forming counterions (*e.g*., sodium), etc. These and other known pharmaceutical carriers, excipients, and/or additives suitable for use in the formulations described herein are known in the art, for example, as listed in "Remington: The Science & Practice of Pharmacy", 21st ed., Lippincott Williams & Wilkins (2005), and "Physician's Desk Reference", 60th ed., Medical Economics, Montvale, New Jersey (2005). The suitable pharmaceutically acceptable carrier may be routinely selected based on the desired or required mode of administration, solubility, and/or stability.

In the present application, the term "mix" or "mixing" generally refers to a process of combining one or more compounds, cells, molecules, etc., together in the same area. This process may be performed, for example, in a test tube, a culture dish, or any container that allows for the mixing of one or more compounds, cells, or molecules.

In the present application, the term "...occurring" generally means that a patient or observed individual shows clinical signs related to a specific disease; for example, in the present application, it may refer to showing clinical signs related to an early, acute hematogenous or chronic prosthetic joint infection.

In the present application, the term "comprise" or "comprising" generally refers to the inclusion of the explicitly specified features without excluding other elements. The terms "not less than" and "not more than" generally refer to situations where the number itself is included.

In the present application, the term "selected from" generally refers to the inclusion of the selected objects and all combinations thereof. For example, "selected from (:) A, B, and C" refers to the inclusion of all combinations of A, B, and C, e.g., A, B, C, A+B, A+C, B+C, or A+B+C.

In the present application, the terms "effective amount" and "effective dose" are used interchangeably and generally refer to an amount sufficient to achieve, or at least partially achieve, the desired therapeutic effect. When used for a patient in need, it generally refers to an amount sufficient to cure a patient already suffering from a disease, or at least partially prevent the disease and complications thereof. The effective amount for this use will depend on the severity of the infection in the patient and the overall state of the patient's own immune system. In the present application, the term "co-administration" generally refers to administration of the therapeutic agent described herein with an additional therapeutic agent or drug as a single formulation or as multiple separate formulations. The co-administration may be simultaneous or sequential in any order; for example, it may preferably last for a certain period of time when both (or all) pharmaceutically active ingredients simultaneously exert their biological activities. The therapeutic agent of the present application and the additional therapeutic agent are not necessarily administered via the same route of administration.

In the present application, the term "about" or "approximately" generally refers to an acceptable error range for a specific value as determined by one of ordinary skill in the art, which depends in part on how the value is measured or determined, i.e., the limitations of the measurement system. For example, "about" or "approximately" may mean being within 1 or more than 1 standard deviation as per the practice in the art. Alternatively, "about" or "approximately" may mean a range of up to 10% or 20% (i.e., ±10% or ±20%). For example, about 3 mg may include any quantity between 2.7 mg and 3.3 mg (for 10%) or between 2.4 mg and 3.6 mg (for 20%). Furthermore, particularly with respect to biological systems or processes, the term may mean a value of up to an order of magnitude or a value of up to 5-fold. Unless otherwise specified, when a specific value or composition is provided in this specification and the appended claims, the meaning of "about" or "approximately" should be assumed to be within an acceptable error range for the specific value or composition.

### Detailed Description of the Invention

In one aspect, the present application provides a method for treating prosthetic joint infections (PJI), which may comprise administering a therapeutic agent to the joint cavity of a patient in need, wherein the therapeutic agent may comprise a compound of formula (I) and/or a pharmaceutically acceptable salt, a hydrate, a solvate, a prodrug, a metabolite, or a deuteride thereof.

In some embodiments, the method may comprise administering the therapeutic agent to the joint cavity of the patient in need with a dosing frequency optionally selected from: once daily, once every 2 days, once every 3 days, once every 4 days, once every 5 days, once every 6 days, and once every 7 days.

In some embodiments, the method may comprise administering the therapeutic agent to the joint cavity of the patient in need for a duration of 1 day to about 6 months, wherein during the duration of 1 day to about 6 months, the dosing frequency of the therapeutic agent may remain the same or may be changed. For example, the method may comprise administering the therapeutic agent to the joint cavity of the patient in need for a duration optionally selected from: 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 26 days, 27 days, 28 days, 29 days, 30 days (1 month), 31 days, 32 days, 33 days, 34 days, 35 days, 36 days, 37 days, 38 days, 39 days, 40 days, 41 days, 42 days, 43 days, 44 days, 45 days, 46 days, 47 days, 48 days, 49 days, 50 days, 51 days, 52 days, 53 days, 54 days, 55 days, 56 days, 57 days, 58 days, 59 days, 60 days (2 months), about 3 months, about 4 months, about 5 months, and about 6 months, wherein during the duration of continuous administration of the therapeutic agent, the frequency of administration of the therapeutic agent may remain the same, or may be changed according to the patient's actual needs or in accordance with medical advice.

In some embodiments, in a single administration, the therapeutic agent is administered to the patient in need via one or more intraarticular injections. For example, in a single administration, the therapeutic agent is administered to the joint cavity of the patient in need via 1, 2, 3, 4, 5, 6, or more than 6 bolus injections, wherein the 2 or more bolus injections may be performed consecutively, or may be performed at identical or different time intervals; the therapeutic agent administered via the 2 or more bolus injections may comprise the same or different amounts of the compound of formula (I) and/or the pharmaceutically acceptable salt, the hydrate, the solvate, the prodrug, the metabolite, or the deuteride thereof.

In some embodiments, in a single administration, the therapeutic agent comprises about 10 mg to about 300 mg of the compound of formula (I) and/or the pharmaceutically acceptable salt, the hydrate, the solvate, the prodrug, the metabolite, or the deuteride thereof. For example, in a single administration, the amount of the compound of formula (I) and/or the pharmaceutically acceptable salt, the hydrate, the solvate, the prodrug, the metabolite, or the deuteride thereof contained in the therapeutic agent may be about: 10 mg, 20 mg, 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, or 300 mg.

In some embodiments, the therapeutic agent further comprises additional components selected from other drugs, pharmaceutically acceptable carriers, solvents, and buffers. For example, the additional components are not mixed with the compound of formula (I) and/or the pharmaceutically acceptable salt, the hydrate, the solvate, the prodrug, the metabolite, or the deuteride thereof. For example, the phrase "not mixed with" may mean that the additional components and the compound of formula (I) and/or the pharmaceutically acceptable salt, the hydrate, the solvate, the prodrug, the metabolite, or the deuteride thereof are separately placed in different containers and/or devices. For example, the phrase "not mixed with" may mean that in the therapeutic agent, the container or device for placing the compound of formula (I) and/or the pharmaceutically acceptable salt, the hydrate, the solvate, the prodrug, the metabolite, or the deuteride thereof, and the container or device for placing the additional components are separately placed in different storage spaces and/or under different storage conditions. For example, the phrase "not mixed with" may mean that the additional components and the compound of formula (I) and/or the pharmaceutically acceptable salt, the hydrate, the solvate, the prodrug, the metabolite, or the deuteride thereof are separately packaged and stored in their respective containers and/or devices in specific measurement units. For example, the phrase "not mixed with" may mean that the additional components are mixed with the compound of formula (I) and/or the pharmaceutically acceptable salt, the hydrate, the solvate, the prodrug, the metabolite, or the deuteride thereof only before the use of the therapeutic agent; this mixing process may comprise using a mixing device (which may or may not be included in the therapeutic agent), transferring the additional components and the compound of formula (I) and/or the pharmaceutically acceptable salt, the hydrate, the solvate, the prodrug, the metabolite, or the deuteride thereof into a container or device containing another component for mixing, and/or transferring both the additional components and the compound of formula (I) and/or the pharmaceutically acceptable salt, the hydrate, the solvate, the prodrug, the metabolite, or the deuteride thereof into an additional container or device for mixing.

In some embodiments, the therapeutic agent comprises additional components selected from other drugs, pharmaceutically acceptable carriers, solvents, and buffers, and one or more of the additional components are mixed with the compound of formula (I) and/or the pharmaceutically acceptable salt, the hydrate, the solvate, the prodrug, the metabolite, or the deuteride thereof. For example, the phrase "mixed with" may mean that one or more of the additional components and the compound of formula (I) and/or the pharmaceutically acceptable salt, the hydrate, the solvate, the prodrug, the metabolite, or the deuteride thereof are mixed and placed together in the same container or device. For example, the phrase "mixed with" may mean that one or more of the additional components and the compound of formula (I) and/or the pharmaceutically acceptable salt, the hydrate, the solvate, the prodrug, the metabolite, or the deuteride thereof are mixed, and then packaged and stored in the same or different containers or devices in specific measurement units. For example, the phrase "mixed with" may mean that after one or more of the additional components are mixed with the compound of formula (I) and/or the pharmaceutically acceptable salt, the hydrate, the solvate, the prodrug, the metabolite, or the deuteride thereof, further thorough mixing (e.g., vigorous shaking to ensure complete mixing) is still required before the therapeutic agent is used.

In some embodiments, the method further comprises administering an effective amount of an additional therapeutic agent to the patient in need. For example, the method further comprises administering an effective amount of an additional therapeutic agent to the patient via injection (mainly referring to non-intraarticular routes, e.g., intravenous injection, intramuscular injection, and/or subcutaneous injection), oral administration, intracavitary administration (other than intraarticular administration), enteral administration, and/or transdermal absorption. For example, the additional therapeutic agent may be selected from antibacterial agents, anesthetics, analgesics, sedatives, hormonal drugs, anti-inflammatory drugs, anticoagulants, platelet aggregation inhibitors, antipyretics, cell membrane permeabilizers, and muscle relaxants.

Prosthetic joint infection has a complex spectrum of pathogenic bacteria, and may be caused by an infection with a single pathogenic bacterium or a mixture of multiple pathogenic bacteria. In some embodiments, the prosthetic joint infection may be caused by Gram-positive bacteria. For example, the prosthetic joint infections may be caused by *Staphylococcus aureus* and/or coagulase-negative *staphylococcal.* Specifically, the coagulase-negative *staphylococcus* may be selected from *Staphylococcus epidermidis, Staphylococcus saprophyticus, Staphylococcus xylosus, Staphylococcus haemolyticus, Staphylococcus capitis, Staphylococcus warneri, Staphylococcus simulans, Staphylococcus xylosus subsp. xylosus,* and *Staphylococcus simiae.* In the present application, the Gram-positive bacteria may also include a drug-resistant strain thereof; for example, the drug-resistant strain may demonstrate single-drug resistance to rifamycins (e.g., rifampicin), single-drug resistance to quinolones or quinolizinones, or resistance to both rifamycins and quinolizinones. For example, the drug-resistant strain may also be resistant to other antibiotics, including macrolide drugs such as clarithromycin, azithromycin, and roxithromycin; nitroimidazole drugs such as metronidazole, ornidazole, pretomanid, and delamanid; aminoglycoside drugs such as streptomycin and amikacin; β-lactam drugs such as methicillin, ampicillin, and amoxicillin; tetracycline drugs such as tetracycline, tigecycline, and minocycline; oxazolidinone drugs such as linezolid and tedizolid; nitrofuran drugs such as furazolidone; polypeptide drugs such as vancomycin and polymyxin; diarylquinoline drugs such as bedaquiline; clofazimine; etc. In the present application, the diagnosis of whether the patient has prosthetic joint infections can be achieved by diagnostic means and criteria known in the art, combined with the patient's detailed medical history and examination results such as results from physical, serological, microbiological, pathological, and imaging examinations.

In some embodiments, the prosthetic joint infections (PJI) comprise early PJI, acute hematogenous PJI, and chronic PJI. Due to the complexity and variability of disease conditions and pathogenic mechanisms of PJIs, this field has not yet established a unified standard for the diagnosis and classification of PJIs. However, according to the clinical features and the time of infection onset of PJI, classification methods commonly used at present may include the Tsukayama method (Tsukayama DT, Estrada R, Gustilo RB. Infection after total hip arthroplasty. A study of the treatment of one hundred and six infections[J]. J Bone Joint Surg Am, 1996, 78(4): 512-523; and Tsukayama DT, Goldberg VM, Kyle R. Diagnosis and management of infection after total knee arthroplasty[J]. J Bone Joint Surg Am, 2003, 85A (1): 75-80.), the Zimmerli method (Zimmerli W, Trampuz A, Ochsner PE. Prosthetic-joint infections[J]. N Engl J Med, 2004, 351(16): 1645-1654.), the Coventry method (Coventry MB. Treatment of infections occurring in total hip surgery. Orthop Clin North Am 1975; 6:991-1003.), the Barrett method (Barrett L, Atkins B. The clinical presentation of prosthetic joint infection. J Antimicrob Chemother. 2014 Sep; 69 Suppl 1: i25-7.), *etc.* For example, Zimmerli *et al.* defined a PJI occurring within 3 months after an artificial prosthetic joint replacement surgery as an early PJI, a PJI occurring within 3 months to 24 months after the surgery as a delayed or intermediate PJI, and a PJI occurring more than 24 months after the surgery as a late infection. For example, the prosthetic joint infections may also be acute hematogenous PJI occurring within about 4 weeks after the surgery.

In some embodiments, the patient in need may comprise a subject who has not yet developed the prosthetic joint infections (PJI) but is at risk of developing the PJI. For example, the method may comprise administering the therapeutic agent to the joint cavity of the subject for a duration of 1 day to 14 days. Specifically, the method may comprise administering the therapeutic agent to the joint cavity of the subject for a duration of 1 day, 2 days, 3 days, 4 days, 5 days, 6 days, 7 days, 8 days, 9 days, 10 days, 11 days, 12 days, 13 days, or 14 days, wherein during the duration, the frequency of administration of the therapeutic agent may remain the same, or may be changed according to the subject's actual needs or in accordance with medical advice. For example, the first day of the duration of 1 day to 14 days is the day on which the subject undergoes the prosthetic joint replacement surgery. In the present application, whether the subject is at risk of developing the PJI can be determined based on the subject's detailed medical history, physical examination, diagnostic report, treatment plan, *etc.*

In another aspect, the present application provides use of the compound of formula (I) and/or the pharmaceutically acceptable salt, the hydrate, the solvate, the prodrug, the metabolite, or the deuteride thereof in the preparation of a therapeutic agent, wherein the therapeutic agent is used for treating prosthetic joint infections, and the therapeutic agent is formulated to be suitable for intraarticular administration to a patient in need.

In some embodiments, the therapeutic agent is formulated to be suitable for administration to the joint cavity of the patient in need with a dosing frequency selected from: once daily, once every 2 days, once every 3 days, once every 4 days, once every 5 days, once every 6 days, and once every 7 days.

In some embodiments, the therapeutic agent is formulated to be suitable for administration to the joint cavity of the patient in need for a duration of 1 day to about 6 months, wherein during the duration of 1 day to about 6 months, the therapeutic agent is formulated to be suitable for administration to the patient in need at the same or different dosing frequencies. For example, the therapeutic agent may be formulated to be suitable for administration to the joint cavity of the patient in need for a duration optionally selected from: 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 26 days, 27 days, 28 days, 29 days, 30 days (1 month), 31 days, 32 days, 33 days, 34 days, 35 days, 36 days, 37 days, 38 days, 39 days, 40 days, 41 days, 42 days, 43 days, 44 days, 45 days, 46 days, 47 days, 48 days, 49 days, 50 days, 51 days, 52 days, 53 days, 54 days, 55 days, 56 days, 57 days, 58 days, 59 days, 60 days (2 months), about 3 months, about 4 months, about 5 months, and about 6 months, wherein during the duration, the therapeutic agent is formulated to be suitable for administration to the patient in need at the same dosing frequency, or it may be formulated to be suitable for administration to the patient in need at different dosing frequencies according to the patient's actual needs or in accordance with medical advice.

In some embodiments, in a single administration, the therapeutic agent is formulated to be suitable for one or more intraarticular injections. For example, in a single administration, the therapeutic agent is formulated to be suitable for administration to the joint cavity of the patient in need via 1, 2, 3, 4, 5, 6, or more than 6 bolus injections, wherein for the 2 or more bolus injections, the therapeutic agent can be formulated to be suitable for consecutive bolus injections, or the therapeutic agent can be formulated to be suitable for bolus injections at identical or different time intervals; the therapeutic agent administered via the 2 or more bolus injections may comprise the same or different amounts of the compound of formula (I) and/or the pharmaceutically acceptable salt, the hydrate, the solvate, the prodrug, the metabolite, or the deuteride thereof.

In some embodiments, in a single administration, the therapeutic agent comprises about 10 mg to about 300 mg of the compound of formula (I) and/or the pharmaceutically acceptable salt, the hydrate, the solvate, the prodrug, the metabolite, or the deuteride thereof. For example, in a single administration, the amount of the compound of formula (I) and/or the pharmaceutically acceptable salt, the hydrate, the solvate, the prodrug, the metabolite, or the deuteride thereof contained in the therapeutic agent may be about: 10 mg, 20 mg, 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, or 300 mg.

In some embodiments, the therapeutic agent further comprises additional components selected from other drugs, pharmaceutically acceptable carriers, solvents, and buffers. For example, the additional components are not mixed with the compound of formula (I) and/or the pharmaceutically acceptable salt, the hydrate, the solvate, the prodrug, the metabolite, or the deuteride thereof. For example, the phrase "not mixed with" may mean that the additional components and the compound of formula (I) and/or the pharmaceutically acceptable salt, the hydrate, the solvate, the prodrug, the metabolite, or the deuteride thereof are separately placed in different containers and/or devices. For example, the phrase "not mixed with" may mean that in the therapeutic agent, the container or device for placing the compound of formula (I) and/or the pharmaceutically acceptable salt, the hydrate, the solvate, the prodrug, the metabolite, or the deuteride thereof, and the container or device for placing the additional components are separately placed in different storage spaces and/or under different storage conditions. For example, the phrase "not mixed with" may mean that the additional components and the compound of formula (I) and/or the pharmaceutically acceptable salt, the hydrate, the solvate, the prodrug, the metabolite, or the deuteride thereof are separately packaged and stored in their respective containers and/or devices in specific measurement units. For example, the phrase "not mixed with" may mean that the additional components are mixed with the compound of formula (I) and/or the pharmaceutically acceptable salt, the hydrate, the solvate, the prodrug, the metabolite, or the deuteride thereof only before the use of the therapeutic agent; this mixing process may comprise using a mixing device (which may or may not be included in the therapeutic agent), transferring the additional components and the compound of formula (I) and/or the pharmaceutically acceptable salt, the hydrate, the solvate, the prodrug, the metabolite, or the deuteride thereof into a container or device containing another component for mixing, or transferring both the additional components and the compound of formula (I) and/or the pharmaceutically acceptable salt, the hydrate, the solvate, the prodrug, the metabolite, or the deuteride thereof into an additional container or device for mixing.

In some embodiments, the therapeutic agent comprises additional components selected from other drugs, pharmaceutically acceptable carriers, solvents, and buffers, and one or more of the additional components are mixed with the compound of formula (I) and/or the pharmaceutically acceptable salt, the hydrate, the solvate, the prodrug, the metabolite, or the deuteride thereof. For example, the phrase "mixed with" may mean that one or more of the additional components and the compound of formula (I) and/or the pharmaceutically acceptable salt, the hydrate, the solvate, the prodrug, the metabolite, or the deuteride thereof are mixed and placed together in the same container or device. For example, the phrase "mixed with" may mean that one or more of the additional components and the compound of formula (I) and/or the pharmaceutically acceptable salt, the hydrate, the solvate, the prodrug, the metabolite, or the deuteride thereof are mixed, and then packaged and stored in the same or different containers or devices in specific measurement units. For example, the phrase "mixed with" may mean that after one or more of the additional components are mixed with the compound of formula (I) and/or the pharmaceutically acceptable salt, the hydrate, the solvate, the prodrug, the metabolite, or the deuteride thereof, further thorough mixing (e.g., vigorous shaking to ensure complete mixing) is still required before the therapeutic agent is used.

In some embodiments, the therapeutic agent is formulated to be suitable for co-administration with an effective amount of an additional therapeutic agent to the patient in need. For example, the therapeutic agent may be formulated to be suitable for co-administration with an effective amount of an additional therapeutic agent to the patient in need, wherein the additional therapeutic agent may be administered to the patient via injection (mainly referring to non-intraarticular routes, e.g., intravenous injection, intramuscular injection, and/or subcutaneous injection), oral administration, intracavitary administration (other than intraarticular administration), enteral administration, and/or transdermal absorption. For example, the additional therapeutic agent may be selected from antibacterial agents, anesthetics, analgesics, sedatives, hormonal drugs, anti-inflammatory drugs, anticoagulants, platelet aggregation inhibitors, antipyretics, cell membrane permeabilizers, and muscle relaxants.

In some embodiments, the prosthetic joint infections (PJI) are caused by Gram-positive bacterial infections. For example, the prosthetic joint infection may be caused by *Staphylococcus aureus* and/or a coagulase-negative *staphylococcal.* Specifically, the coagulase-negative *staphylococcus* may be selected from *Staphylococcus epidermidis, Staphylococcus saprophyticus, Staphylococcus xylosus, Staphylococcus haemolyticus, Staphylococcus capitis, Staphylococcus warneri, Staphylococcus simulans, Staphylococcus xylosus subsp. xylosus,* and *Staphylococcus simiae.* In the present application, the Gram-positive bacteria may also include a drug-resistant strain thereof; for example, the drug-resistant strain may demonstrate single-drug resistance to rifamycins (e.g., rifampicin), single-drug resistance to quinolones or quinolizinones, or resistance to both rifamycins and quinolizinones. For example, the drug-resistant strain may also be resistant to other antibiotics, including macrolide drugs such as clarithromycin, azithromycin, and roxithromycin; nitroimidazole drugs such as metronidazole, ornidazole, pretomanid, and delamanid; aminoglycoside drugs such as streptomycin and amikacin; β-lactam drugs such as methicillin, ampicillin, and amoxicillin; tetracycline drugs such as tetracycline, tigecycline, and minocycline; oxazolidinone drugs such as linezolid and tedizolid; nitrofuran drugs such as furazolidone; polypeptide drugs such as vancomycin and polymyxin; diarylquinoline drugs such as bedaquiline; clofazimine; etc. In the present application, the diagnosis of whether the patient in need has a prosthetic joint infection can be achieved by diagnostic means and criteria known in the art, combined with the patient's detailed medical history and examination results such as results from physical, serological, microbiological, pathological, and imaging examinations.

In some embodiments, the prosthetic joint infections (PJI) comprise early PJI, acute hematogenous PJI, and chronic PJI. For example, a PJI occurring within 3 months after an artificial prosthetic joint replacement surgery is defined as an early PJI, a PJI occurring within 3 months to 24 months after the surgery is defined as a delayed or intermediate PJI, and a PJI occurring more than 24 months after the surgery is defined as a late infection (Zimmerli *et al.,* 2004). For example, the prosthetic joint infection may be an acute hematogenous PJI occurring within about 4 weeks after the surgery.

In some embodiments, the patient in need may comprise a subject who has not yet developed the prosthetic joint infections (PJI) but is at risk of developing the PJI. For example, the therapeutic agent may be formulated to be suitable for administration to the joint cavity of the subject for a duration of 1 day to 14 days. Specifically, the therapeutic agent may be formulated to be suitable for administration to the joint cavity of the subject for a duration optionally selected from: 1 day, 2 days, 3 days, 4 days, 5 days, 6 days, 7 days, 8 days, 9 days, 10 days, 11 days, 12 days, 13 days, and 14 days, wherein during the duration, the therapeutic agent is formulated to be suitable for administration to the joint cavity of the subject at the same dosing frequency, or it may be formulated to be suitable for administration to the joint cavity of the subject at different dosing frequencies according to the subject's actual needs or in accordance with medical advice. For example, the first day of the duration of 1 day to 14 days is the day on which the subject undergoes the prosthetic joint replacement surgery. In the present application, whether the subject is at risk of developing the PJI can be determined based on the subject's detailed medical history, physical examination, diagnostic report, treatment plan, etc.

Without being bound by any theory, the following examples are intended only to illustrate the methods, use, *etc.* of the present application, but not to limit the scope of the invention of the present application.

### Examples

### Example 1

### Evaluation of Efficacy of Treatment Method of the Present Application in Mouse Model

### 1. Experimental method

In this example, female mice aged 8-10 weeks were used and randomly grouped. Before the start of drug administration treatment, each mouse underwent a surgery involving the placement of a metal implant into the femoral-tibial joint and an intraarticular inoculation of methicillin-sensitive *Staphylococcus aureus* (MSSA, ATCC6538 strain) at the surgical site.

Therapeutic agents were formulated as follows:
(1) compound of formula (I): when used as a therapeutic agent for intraarticular injection (IA), the compound was dissolved in 5% dextrose in water (D5W) and formulated to the required concentrations; when used as a therapeutic agent for intraperitoneal injection (IP), the compound was dissolved in a solvent system of ethanol/NaHCO₃/6% Cremaphor and formulated to the required concentrations; (2) vancomycin: the drug was dissolved in sterile water for injection (WFI) and formulated to the required concentration to obtain a therapeutic agent for intraarticular injection.

The experimental design for evaluating the therapeutic effect was as follows:

| **Mouse group** | **Treatment** | **Drug dose (mg/kg)** | **Route of administration** | **Treatment regimen** | **Time of administration** | **Sampling day** |
|---|---|---|---|---|---|---|
| 1 | TNP-2092 | 45 | IA | QD × 7 days | Day 7 | Day 14 |
| 2 | | 15 | IA | QD × 7 days | Day 7 | Day 14 |
| 3 | | 5 | IA | QD × 7 days | Day 7 | Day 14 |
| 4 | | 45 | IP | BID × 7 days | Day 7 | Day 14 |
| 5 | | 15 | IP | BID × 7 days | Day 7 | Day 14 |
| 6 | | 5 | IP | BID × 7 days | Day 7 | Day 14 |
| 7 | Vancomycin | 45 | IA | QD × 7 days | Day 7 | Day 14 |
| 8 | D5W vehicle | No drug | IA | QD × 7 days | Day 7 | Day 14 |
| 9 | Infection control | No drug | N/A | N/A | N/A | Day 14 |
| 10 | | No drug | N/A | N/A | N/A | Day 7 |

| | | | | | | |
|---|---|---|---|---|---|---|
| Note: IA: intraarticular injection, IP: intraperitoneal injection, QD: once daily, BID: twice daily. | | | | | | |

### 2. Analysis of results

Samples were obtained from the surgical and inoculation sites of the mice, and colony counts are performed on the agar plates. The number of colonies converted to CFU/tissue by multiplying the number of colonies by the volume of the homogenate spotted and the dilution at which the colonies were counted (5-50 colonies/spot). The resulting value was expressed as log₁₀ CFU/tissue and used to calculate the mean and standard deviations (the results are shown in Table 1).

**Table 1. Analysis data of colony counts from mouse pin and femur**

| **Test content** | **Dose (mg/kg)** | **Treatment** | | **Days** | **Pin** | | **Femur** | | **Log₁₀ CFU Reduction vs. day 14 control** | |
|---|---|---|---|---|---|---|---|---|---|---|
| | | | | | **Mean Log₁₀ CFU** | **Standard deviation** | **Mean Log₁₀ CFU** | **Standard deviation** | | |
| | | **Volume/route of administration** | **Administration regimen** | | | | | | **Pin** | **Femur** |
| TNP-2092 | 45 | 25 µL IA | QD x 7 days | 14 | 2.05 | 0.00 | 3.46 | 1.18 | -2.72 | -3.42 |
| | 15 | | | | 2.23 | 0.53 | 3.76 | 0.82 | -2.54 | -3.12 |
| | 5 | | | | 2.27 | 0.54 | 5.17 | 0.52 | -2.50 | -1.71 |
| TNP-2092 | 45 | 0.2 mL IP | BID x 7 days | | 2.05 | 0.00 | 3.42 | 0.85 | -2.72 | -3.46 |
| | 15 | | | | 2.52 | 0.52 | 5.33 | 0.77 | -2.25 | -1.55 |
| | 5 | | | | 4.26 | 1.10 | 6.51 | 0.56 | -0.51 | -0.37 |
| Vancomycin | 45 | 25 µL IA | QD x 7 days | | 3.49 | 0.80 | 6.08 | 0.58 | -1.28 | -0.80 |
| IA vehicle | N/A | 25 µL IA | QD x 7 days | 14 | 4.93 | 0.63 | 6.94 | 0.30 | 0.16 | 0.06 |
| Infection control | N/A | N/A | N/A | 14 | 4.77 | 0.77 | 6.88 | 0.79 | 0.00 | 0.00 |
| | N/A | N/A | N/A | 7 | 5.35 | 0.55 | 6.77 | 0.28 | 0.58 | -0.11 |

The results in Table 1 and FIG. 1A-1B show that on day 7 after the start of treatment, the mean colony counts for the pin and femur were 5.35 log₁₀ CFU and 6.77 log₁₀ CFU, respectively. On day 14, the corresponding counts for the pin and femur in the untreated control animals were 4.77 log₁₀ CFU and 6.88 log₁₀ CFU, respectively.

The intraarticular administration of the compound of formula (I) exhibited mean pin colony counts and mean femur colony counts that were 2.5-2.72 log₁₀ CFU and 1.71-3.42 log₁₀ CFU lower than those in the untreated control animals, respectively. Compared to the control group, the results of all IA dose groups of the compound of formula (I) were considered statistically significant (P < 0.0001). Compared to the IA dose group of vancomycin, the pin colony counts in the 45 mg/kg, 15 mg/kg, and 5 mg/kg IA dose groups of the compound of formula (I), as well as the femur colony counts in the 45 mg/kg and 15 mg/kg IA dose groups of the compound of formula (I), were also determined to be statistically significant (P < 0.0001). Compared to the IP administration (BID) of the compound of formula (I), although the 45 mg/kg IA dose showed a similar therapeutic effect, the femur colony counts in the 15 mg/kg and 5 mg/kg IA dose groups (QD) of the compound of formula (I) were also determined to be statistically significant (P < 0.0001) compared to the IP administration of the compound of formula (I) at the same doses, and the pin colony counts in the 15 mg/kg IA dose group of the compound of formula (I) were also determined to be statistically significant (P < 0.0001) compared to the 15 mg/kg IP dose group of the compound of formula (I). Moreover, the intraarticular administration had a lower dosing frequency, providing an advantage in improving patient compliance.

### Example 2

### Evaluation of Efficacy of Treatment Method of the Present Application in Rat PJI Model

### 1. Experimental method

In this example, rats aged about 17 weeks were used. Before the start of drug administration treatment, each rat underwent an aseptic surgery to insert an artificial prosthesis into the knee joint, followed by an intraarticular inoculation of methicillin-resistant *Staphylococcus aureus* (MRSA, ATCC43300 strain) at the surgical site.

Meanwhile, the experimental groups and their experimental content settings were specifically designed as shown in the table below:

| **Group** | **Number of animals** | **Administration dose (mg/kg)** | **Treatment duration** | **Time of administratio n** | **Sample collectio n** | **Test indicators** |
|---|---|---|---|---|---|---|
| Blank group C7 | 5 | N/A | N/A | N/A | D7 | CFU count of samples, periarticular tissue pathology, scanning electron microscopy observation of prosthesis |
| Vehicle group Con | 10 | N/A | QD × 14 days | D7 | D21 | |
| Vancomycin Van | 10 | 44 | | | | |
| Compound of formula (I) Low-dose group T2.5 | 10 | 2.5 | | | | |
| Compound of formula (I) Medium-dose group T5 | 10 | 5 | | | | |
| Compound of formula (I) High-dose group T10 | 10 | 10 | | | | |

| | | | | | | |
|---|---|---|---|---|---|---|
| Note: The compound of formula (I) was formulated to the required concentrations (for the low-, medium-, and high-dose groups) with 5% dextrose in water (D5W) on the day of use; vancomycin (clinically effective dose, equivalent to an effective dose for rats of 44 mg/kg) was formulated to the required concentration with sterile water for injection. | | | | | | |

### 2. Analysis of results

In this example, the MIC values (minimum inhibitory concentrations) of vancomycin and the compound of formula (I) against the MRSA ATCC43300 strain were determined, obtaining an MIC_{vancomycin} of 0.5 µg/mL and an MIC_{formula (I)} of 0.032 µg/mL, indicating that the compound of formula (I) had a stronger *in vitro* bactericidal effect compared to vancomycin. As can be seen from the electron microscope results shown in FIG. 2A, a large number of colonies and biofilms formed on the prosthesis in the control infection group (vehicle group); in the low-dose group of the compound of formula (I), the formation of a relatively large number of colonies could still be observed, with relatively abundant microscopic proteins surrounding the bacteria; in the medium-dose group, the microscopic proteins showed a reticular distribution, partially aggregated into bundles, with individual bacteria still present; in the high-dose group, the microscopic proteins were evenly distributed, with individual cocci embedded therein; in the vancomycin treatment group, a relatively large number of bacteria still remained, and the microscopic proteins showed a reticular distribution. The above results show that for the bacteria colonizing the prosthesis, the medium- and high-dose treatment groups of the compound of formula (I) had a stronger ability to inhibit bacterial growth and biofilm formation compared to the vancomycin group.

The pathological H&E staining results of the synovial soft tissue shown in FIG. 2B indicate that the synovium of the control infection group exhibited severe interstitial cell proliferation, an increased number of cell layers, and inflammatory cell infiltration. In contrast, the synovitis symptoms showed different degrees of amelioration in the groups treated with vancomycin and the low, medium, and high doses of the compound of formula (I). Among them, the high-dose group of the compound of formula (I) and the vancomycin group exhibited a significant reduction in inflammatory cell infiltration in the synovium and a remarkable alleviation of synovitis symptoms.

Table 2 shows the statistics of CFU values (tissue bacterial load) of samples taken from the implanted joint prosthesis (primary evaluation site for PJI infections), bone tissue (primary evaluation site for PJI infections), and soft tissue (non-primary evaluation site for PJI infection) after treatment in each group. The pharmacodynamic experimental results indicate that compared to the vehicle control group, the low-, medium-, and high-dose groups of the compound of formula (I) all exhibited good bactericidal effects; compared to the vancomycin group, the low-, medium-, and high-dose groups of the compound of formula (I) exhibited superior bactericidal effects in both the prosthetic and bone tissues; the medium- and high-dose groups of the compound of formula (I) exhibited comparable bactericidal effects to the vancomycin group in the soft tissue.

**Table 2. Prosthesis CFU count results**

| **Group** | **Strain** | **Tissue bacterial load in prosthesis (CFU)** | **Tissue bacterial load in bone tissue (CFU)** | **Tissue bacterial load in soft tissue (CFU)** |
|---|---|---|---|---|
| C7 | S. aureus ATCC43300 2×10⁷CFU/mL | 7.90±2.05×10⁵ | 1.35±0.69×10⁶ | 1.27±0.49×10⁶ |
| Con | | 1.14±0.49×10⁶ | 1.75±0.88×10⁶ | 1.32±0.84×10⁶ |
| T2.5 | | 550±207 *** | 7083±6280 *** | 9717±1387*** |
| T5 | | 0 *** | 1383±1068*** | 5433±3194*** |
| T10 | | 0 *** | 2400±1655*** | 1517±1011*** |
| Van | | 1783±1136 *** | 11733±8583*** | 1050±1174*** |

| | | | | |
|---|---|---|---|---|
| Note: *** represents p < 0.001 compared to the control group (Con), indicating a highly significant difference, as analyzed using the one-way ANOVA method. | | | | |

Summary: In this example, *S. aureus* ATCC43300 was used to establish a stable rat prosthetic joint infection model to evaluate the antibacterial activity of the intraarticular injection of the compound of formula (I). The results show that the intraarticular administration of the compound of formula (I) could effectively reduce the bacterial load in the intraarticular tissues of rats, exhibiting dose-dependent bactericidal activity; compared to the vancomycin treatment group, the high- and medium-dose groups of the compound of formula (I) demonstrated stronger bactericidal ability.

### Example 3

### Pharmacokinetic Analysis of Treatment Method of the Present Application in Male and Female Beagle Dogs

In this example, an experiment with an animal model of male and female beagle dogs was used to evaluate the pharmacokinetic behavior after a single intraarticular injection of the compound of formula (I) in the knee. A total of 6 beagle dogs (3 males and 3 females) were used for this experiment. The experimental animals received a single intraarticular injection of 3 mg/kg of the compound of formula (I) in the left joint, and a single intraarticular injection of 0.1 mL/kg of the vehicle (injection solution of 5% dextrose in water) in the right joint. From the experimental animals, plasma samples were collected before administration (0 hour) and at 0.25 hour (15 minutes), 3 hours, 9 hours, 12 hours, 24 hours, 36 hours, 48 hours, and 72 hours after administration, and left knee synovial fluid samples were collected at 12 hours, 36 hours, and 60 hours after administration. The concentrations of the compound of formula (I) in the plasma and synovial fluid samples were determined using high-performance liquid chromatography-tandem mass spectrometry (LC-MS/MS).

**Table 3. Pharmacokinetic parameters of single intraarticular injection in male and female beagle dogs**

| **Route of administration** | **Intraarticular injection** | |
|---|---|---|
| **Administration dose (mg/kg)** | **3** | |
| **Base** | **Plasma** | **Synovial fluid** |
| **Pharmacokinetic parameter** | **Mean** | **Mean** |
| **Cₘₐₓ (ng/mL)** | 736 | 77400 |
| **Tₘₐₓ (h)** | 24.0 | 12.0 |
| **T_{1/2} (h)** | 12.0 | 6.81 |
| **AUC₀₋ₗₐₛₜ (ng·h/mL)** | 20800 | 1000000 |
| **AUC ratio** | N/A | 46.1 |

| | | |
|---|---|---|
| Note: (1) Cₘₐₓ: peak concentration, Tₘₐₓ: time to peak, T_{1/2}: elimination half-life, AUC: systemic drug exposure, *i.e.,* area under the plasma concentration-time curve, AUC₀₋ₗₐₛₜ: area under the plasma concentration-time curve from time 0 to the last quantifiable time point; (2) AUC ratio = AUC₀₋ₗₐₛₜ in synovial fluid/AUC₀₋ₗₐₛₜ in plasma, N/A: not applicable. | | |

According to the experimental results shown in Table 3, after the aforementioned injection regimen was administered into the joint cavity of beagle dogs, the peak concentrations (Cₘₐₓ) of the compound of formula (I) in plasma and synovial fluid were 736±247 ng/mL and 77400±81900 ng/mL, respectively, which were far higher than its MIC₉₀ values against *Staphylococcus aureus* (15 ng/mL) and *Staphylococcus epidermidis* (8 ng/mL). The concentration of the drug in the synovial fluid was far higher than its biofilm bactericidal activity MBBC₉₀ values (2000 ng/mL and 250 ng/mL) against *Staphylococcus aureus* and *Epidermophyton.* Moreover, the drug had relatively high exposure in both plasma and synovial fluid, with AUC₀₋ₗₐₛₜ values of 20800±7150 ng•h/mL and 1000000±1050000 ng•h/mL, respectively; the AUC₀₋ₗₐₛₜ ratio of synovial fluid to plasma was 46.1, indicating that the drug can be concentratedly distributed in the synovial fluid, which is expected to reduce the clinical side effects caused by the relatively high systemic exposure.

## Claims

1. A method for preventing or treating prosthetic joint infections (PJI) or alleviating PJI-related symptoms, comprising administering a therapeutic agent to a joint cavity of a patient in need, wherein the therapeutic agent comprises a compound of formula (I) and/or a pharmaceutically acceptable salt, a hydrate, a solvate, a prodrug, a metabolite, or a deuteride thereof,

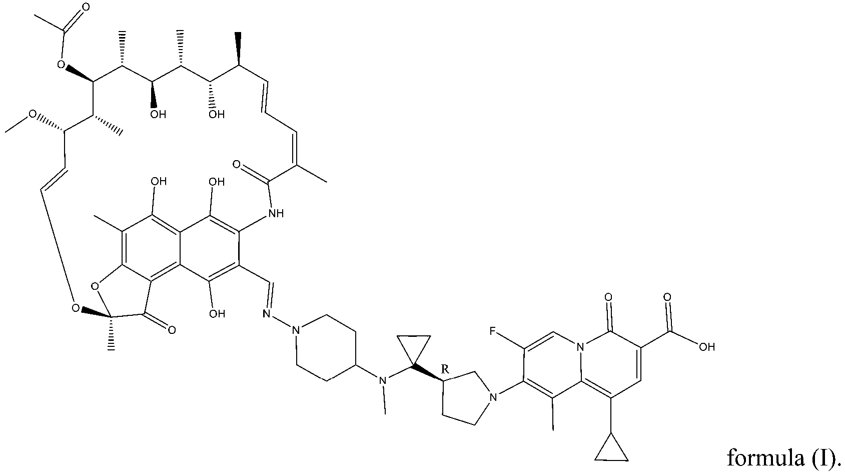

2. The method according to claim 1, comprising administering the therapeutic agent to the joint cavity of the patient in need at a dosing frequency optionally selected from: once daily, once every 2 days, once every 3 days, once every 4 days, once every 5 days, once every 6 days, and once every 7 days.

3. The method according to any one of claims 1-2, comprising administering the therapeutic agent to the joint cavity of the patient in need for a duration of 1 day to about 6 months, wherein during the duration of 1 day to about 6 months, the dosing frequency of the therapeutic agent remains the same or is changed.

4. The method according to any one of claims 1-3, wherein in a single administration, the therapeutic agent comprises about 10 mg to about 300 mg of the compound of formula (I) and/or the pharmaceutically acceptable salt, the hydrate, the solvate, the prodrug, the metabolite, or the deuteride thereof.

5. The method according to any one of claims 1-4, wherein the therapeutic agent further comprises additional components selected from other drugs, pharmaceutically acceptable carriers, solvents, and buffers.

6. The method according to claim 5, wherein the additional components are not mixed with the compound of formula (I) and/or the pharmaceutically acceptable salt, the hydrate, the solvate, the prodrug, the metabolite, or the deuteride thereof.

7. The method according to claim 5, wherein one or more of the additional components are mixed with the compound of formula (I) and/or the pharmaceutically acceptable salt, the hydrate, the solvate, the prodrug, the metabolite, or the deuteride thereof.

8. The method according to any one of claims 1-7, further comprising administering an effective amount of an additional therapeutic agent to the patient in need.

9. The method according to any one of claims 1-8, the prosthetic joint infections (PJI) are caused by Gram-positive bacteria.

10. The method according to any one of claims 1-9, wherein the prosthetic joint infections (PJI) are caused by *Staphylococcus aureus* and/or coagulase-negative *staphylococcal.*

11. The method according to any one of claims 1-10, wherein the prosthetic joint infections (PJI) comprise early PJI, acute hematogenous PJI, and chronic PJI.

12. The method according to any one of claims 1-11, wherein the prosthetic joint infections (PJI) are early PJI occurring within about 3 months after the patient in need undergoes a prosthetic joint replacement surgery, or acute hematogenous PJI occurring within about 4 weeks after the surgery.

13. The method according to any one of claims 1-12, wherein the patient in need comprises a subject who has not yet developed the prosthetic joint infections (PJI) but is at risk of developing the PJI.

14. The method according to claim 13, comprising administering the therapeutic agent to the joint cavity of the subject for a duration of 1 day to 14 days.

15. The method according to claim 14, wherein the first day of the duration of 1 day to 14 days is the day on which the subject undergoes the prosthetic joint replacement surgery.
